# EUROPÄISCHE PATENTANMELDUNG

(11) **EP 2 730 221 A1**
(43) Veröffentlichungstag der Anmeldung: **14.05.2014**
(21) Anmeldenummer: 13192501.8
(22) Anmeldetag: 12.11.2013
(51) Int. Cl.: A61B 5/097, A61B 5/08, A62B 9/00, B01D 53/22, A61L 29/00

(54) **Gasprobenleitung und Gasanalysesystem**

(30) Priorität: 12.11.2012 DE 102012220565
(71) Anmelder: bluepoint medical GmbH & Co. KG, 23923 Selmsdorf (DE)
(72) Erfinder: Lindner, Bernd, 23167 Stockelsdorf (DE)
(74) Vertreter: Seemann & Partner

(57) **Zusammenfassung**

Die Erfindung betrifft eine Gasprobenleitung (12) für ein Analysegas, insbesondere Atemgas, zur Leitung eines Analysesgases von einer Analysegasquelle (4) zu einem Gasanalysator (22), die mit einer Analysegasquelle (4) und einem Gasanalysator (22) verbindbar ist. Die Erfindung betrifft ferner ein Gasanalysesystem (10), eine Verwendung sowie ein Verfahren zum Analysieren eines Analysegases.

Die erfindungsgemäße Gasprobenleitung (12) zeichnet sich dadurch aus, dass die Gasprobenleitung (12) als Schlauch aus einem thermoplastischen Polyether-ester Blockcopolymer-Material ausgebildet ist.

## Beschreibung

Die Erfindung betrifft eine Gasprobenleitung für ein Analysegas, insbesondere Atemgas, zur Leitung eines Analysesgases von einer Analysegasquelle zu einem Gasanalysator, die mit einer Analyse-gasquelle und einem Gasanalysator verbindbar ist. Die Erfindung betrifft ferner ein Gasanalysesystem, eine Verwendung sowie ein Verfahren zum Analysieren eines Analysegases.

Messgasanalysatoren wie beispielsweise Kapnographen werden im klinischen Umfeld eingesetzt, um stationär behandelte oder beatmete Patienten zu überwachen. Dabei wird der Gehalt an Kohlenstoffdioxid in der Ausatemluft eines Patienten gemessen und überwacht. Die Überwachung sorgt für eine deutliche Reduktion von Komplikationen insbesondere bei beatmeten Patienten. Auch in der Anästhesie werden sie bei Einsatz von Narkosegeräten eingesetzt.

Auch andere Gase, wie beispielsweise Sauerstoff, Lachgas oder gasförmige Narkosemittel können in diesem Zusammenhang überwacht werden.

Je nach Messtechnik unterscheidet man Hauptstromverfahren und Nebenstrom- bzw. Seitenstromverfahren. Beim Seitenstromverfahren wird eine geringe Menge Luft ständig abgesaugt und über einen dünnen Schlauch als Gasprobenleitung zum Gasanalysator geleitet. Dabei erfolgt die Messung verzögert. Die Schlauchlängen betragen in diesem Fall typischerweise etwa 2 Meter.

Ein Umstand, der die Überwachung erschwert, ist die in der Atemluft enthaltene Feuchtigkeit. Diese schlägt sich als Kondensat an den Wänden des Schlauches mit dem Messgasstrom nieder und muss vom Gasanalysator ferngehalten werden, um diesen nicht zu beschädigen. Hierfür werden insbesondere Wasserfallen eingesetzt, die Kondenswasser auffangen und in einem mit dem Schlauch verbundenen Reservoir speichern. Wenn dieses Volumen bzw. Reservoir aufgefüllt ist, muss die Wasserfalle entleert oder ersetzt werden.

Insbesondere bei einem Nebenstromverfahren oder Seitenstromverfahren ist es außerdem wichtig, dass ein geringes Totvolumen in dem Nebenstromschlauch bzw. der Gasprobenleitung vorhanden ist, was die Überwachung verschlechtern würde. Ein großes Totvolumen führt bei gegebener Absaugrate zu Mischluft aus inspiratorischer und expiratorischer Phase und damit zu einer fehlerhaften Messung.

Es ist auch bekannt, in die Gasprobenleitung einen Trocknungsabschnitt einzusetzen, der aus einem sulfonierten TetrafluorethylenPolymer, beispielsweise Nafion^{®} von DuPont, besteht. Ein solches System ist beispielsweise in US 2009/0088656 A1 offenbart. Das Material Nafion^{®} ist kostspielig, so dass entsprechend ausgestattete Gasprobenleitungen sehr teuer sind. Der Abschnitt der Gasprobenleitung aus Nafion^{®} ist üblicherweise nicht länger als 10 bis 20 cm. Dieses Material ist nicht knickresistent und muss mit einem Stützgewebe umgeben werden.

Demgegenüber liegt der vorliegenden Erfindung die Aufgabe zugrunde, eine Gasprobenleitung zur Verfügung zu stellen, mit der auf kostengünstige Weise ein längerer Betrieb ohne Tausch der Gasprobenleitung möglich ist bzw. ein Ersatz oder Entleeren von Wasserfallen überflüssig wird.

Diese Aufgabe wird durch eine Gasprobenleitung für ein Analysegas, insbesondere Atemgas, zur Leitung eines Analysesgases von einer Analysegasquelle zu einem Gasanalysator, die mit einer Analysegasquelle und einem Gasanalysator verbindbar ist, gelöst, indem die Gasprobenleitung als Schlauch aus einem thermoplastischen Polyether-ester Blockcopolymer-Material ausgebildet ist.

Die Erfindung beruht auf dem Grundgedanken, dass es möglich ist, Nafion^{®} durch ein Material zu ersetzen, aus dem sich Schläuche für entsprechende Anwendungen im medizinischen Bereich herstellen lassen, das ebenfalls eine hohe Wasserdampftransportrate (Moisture Vapour Transmission Rate, MVTR) aufweist. Die im Vergleich zu Nafion^{®} deutlich niedrigere MVTR bei dem erfindungsgemäß verwendeten Polyether-ester Blockcopolymer-Material wird dadurch ausgeglichen, dass nicht nur ein 10 cm bis 20 cm langer Abschnitt, sondern der gesamte Schlauch der Gasprobenleitung aus dem Material hergestellt ist. Dies bedeutet eine um vielfaches größere Oberfläche des atmungsaktiven Materials, so dass die gleiche Trocknungswirkung für den Gasstrom erzielt wird, wie bei den herkömmlichen Gasprobenleitungen mit einem kurzen Nafion^{®}-Schlaucheinsatz, der noch dazu mit einem umliegenden porösen Schlauchgewebeabschnitt gestützt werden muss, weil Nafion^{®} nicht elastisch und damit nicht knickresistent ist, was zu einer Blockade der Leitung führen kann. Das Polyether-ester BlockcopolymerMaterial weist diese nachteiligen mechanischen Eigenschaften nicht auf.

Damit wird auch die Herstellung der Gasprobenleitung signifikant vereinfacht. Zusammen mit den deutlich geringeren Materialkosten im Vergleich zu Nafion^{®} ergibt sich eine Gasprobenleitung, die bei vergleichbarer Trocknungswirkung für das Analysegas deutlich geringere Herstellungs- und Materialkosten mit sich bringt.

Ein weiterer Vorteil ist, dass die gesamte Gasprobenleitung aus dem atmungsaktiven Material besteht, so dass kondensierte Wassertröpfchen aus der Atemfeuchtigkeit eine sehr große Oberfläche haben, um durch das Wandmaterial des Schlauches nach außen transportiert zu werden. Dies ist ein Prozess, der sehr langsam vonstatten geht, im Vergleich zur Diffusion von gasförmigem Wasserdampf, die als einzelne Moleküle in das atmungsaktive Material eintreten. Ferner entfallen damit Übergänge zwischen verschiedenen Schlauchabschnitten, die weitere Schwachstellen des Schlauchs bilden können.

Vorzugsweise weist das Polyether-ester Blockcopolymer harte Segmente aus Polybutylenterephthalat und weiche Segmente aus einem, insbesondere langkettigen, Polyetherglykol auf. Ein solches Material ist beispielsweise unter dem Handelsnamen Hytrel^{®} von DuPont erhältlich in verschiedenen Härtegraden und mechanischen und chemischen Eigenschaften, sowie von anderen Herstellern unter anderen Handelsnamen, so dass geeignete Materialien für den vorliegend genannten Zweck zur Verfügung stehen.

Die harten Segmente des Copolymers bilden Kristallisationskerne und bestimmen die Härte und die mechanischen Eigenschaften des Materials wie seine Stabilität wesentlich, während die weichen Segmente für den Transport der Wassermoleküle durch das Copolymer-Material verantwortlich sind.

Für eine ausgewogene Mischung aus mechanischer Festigkeit und Flexibilität sowie hoher Wasserdampfpermeabilität treten die Polyethersegmente und die Polyestersegmente des Polyether-ester Blockcopolymers in einem Verhältnis von zwischen 25:75 und 75:25, insbesondere zwischen 40:60 und 60:40, auf.

Um die Menge des in einem Seitenstromverfahren auftretenden Wasserdampfes im abgezweigten Analysegas abführen zu können, ist vorzugsweise vorgesehen, dass der Schlauch bei 38°C und 90% relativer Luftfeuchtigkeit eine Wasserdampftransmissionsrate von mehr als 300 g/m²/24h, insbesondere mehr als 1.000 g/m²/24h, aufweist. Mit entsprechenden Wasserdampftransmissionsraten, die sowohl vom Material selber als auch von der Wanddicke abhängen, ist die anfallende Menge an Feuchtigkeit im Analysegas mit der als Schlauch ausgebildeten Gasprobenleitung in der Regel zu einem ausreichenden Grad abführbar.

Um ausreichende mechanische Eigenschaften des Schlauchs zu erhalten, weist das Polyether-ester Blockcopolymer vorzugsweise eine Shore-D-Härte zwischen 30 und 85, insbesondere zwischen 35 und 60, auf. Bei geringerer Härte wird der Schlauch zu weich und es besteht die Gefahr des Einknickens und dadurch eines Abschlusses des inneren Lumens des Schlauches, während bei einer größeren Shore-D-Härte der Schlauch zu rigide wird und Bruchgefahr besteht. In diesem Fall können auch irreversible Knicke entstehen.

Der Schlauch weist vorzugsweise eine Länge von 0,5 bis 5 m auf, insbesondere 2 bis 4 m, insbesondere zwischen 2 m und 3 m. Bei einer kürzeren Länge ist es möglich, dass die für den Wasserdampftransport zur Verfügung stehende Fläche bzw. Innenfläche des Schlauches zu klein wird und nicht mehr genügend Feuchtigkeit aus dem Analysegas abgeführt wird. Bei einer größeren Menge wird der Abstand zwischen Entnahmepunkt des Analysegases und Gasanalysator so groß, dass die zeitliche Verzögerung der Messung störend wird. Die Länge bzw. Konfektionslänge des Schlauchs kann sich auch nach der Verwendung richten, beispielsweise im konkreten Anwendungsfall nach der Entfernung vom Patienten zum Gasanalysator.

Ebenfalls vorzugsweise weist der Schlauch einen Innendurchmesser zwischen 0,25 und 2 mm, insbesondere zwischen 0,5 und 1 mm, sowie eine Wandstärke von 0,2 mm bis 1,0 mm, insbesondere von 0,4 bis 0,6 mm, auf.

Die der Erfindung zugrunde liegende Aufgabe wird auch durch ein Gasanalysesystem mit einem Gasanalysator, insbesondere Kapnograph, sowie einer erfindungsgemäßen, zuvor beschriebenen Gasprobenleitung gelöst. Bei einem solchen Gasanalysesystem gelangt das Analysegas ausreichend getrocknet zum Gasanalysator, wobei die Gasprobenleitung nur selten ausgetauscht werden muss und wenig kostenintensiv ist.

Die der Erfindung zugrunde liegende Aufgabe wird mit gleichem Effekt auch durch eine Verwendung einer erfindungsgemäßen, zuvor beschrieben Gasprobenleitung zum Vortrocknen eines Analysegases für eine Gasanalyse gelöst, das durch die Gasprobenleitung zu einem Gasanalysator, insbesondere Kapnograph, geleitet wird. Schließlich wird die der Erfindung zugrunde liegende Aufgabe ebenfalls durch ein Verfahren zum Analysieren eines Analysegases gelöst, wobei eine Probe eines Analysegases aus einem Analysegasstrom entnommen und durch eine erfindungsgemäße, zuvor beschriebene Gasprobenleitung vorgetrocknet und zu einem Gasanalysator geleitet wird.

Die zu den einzelnen Erfindungsgegenständen genannten Merkmale, Eigenschaften und Vorteile gelten ohne Einschränkung auch für die jeweils anderen Gegenstände, da sie sich aufeinander beziehen.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand eines Ausführungsbeispiels unter Bezugnahme auf die Zeichnung beschrieben, wobei bezüglich aller im Text nicht näher erläuterten erfindungsgemäßen Einzelheiten ausdrücklich auf die Zeichnung verwiesen wird. Es zeigt:

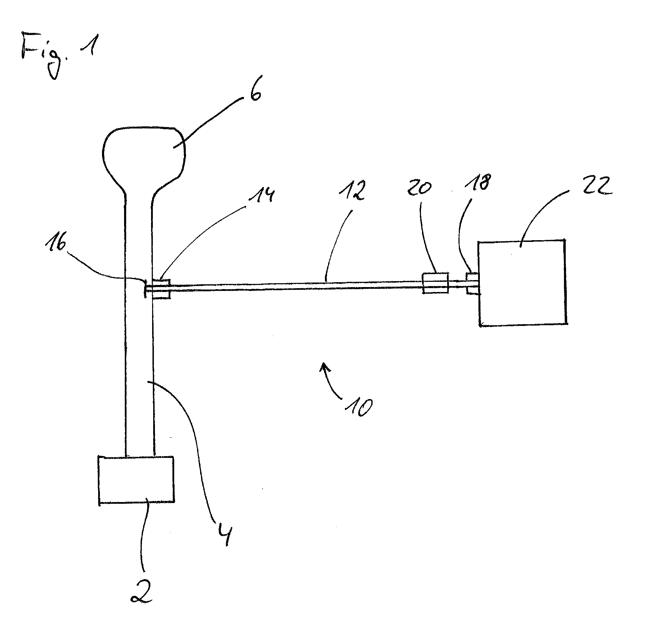

- Fig. 1: eine schematische Darstellung eines erfindungsgemäßen Gasanalysesystems.

Das erfindungsgemäße Gasanalysesystem 10, das in Fig. 1 schematisch dargestellt ist, umfasst eine Gasprobenleitung 12, die einen Anschluss 14 zu einem Atemschlauch 4 aufweist, das ein Beatmungsgerät 2 mit einer Atemmaske 6 für einen Patienten verbinden. Alternativ kann anstelle eines Beatmungsgeräts 2 auch beispielsweise ein Narkosegasgerät angeschlossen sein.

Die Gasprobenleitung 12 ist einstückig bzw. als einstückiger Schlauch ausgebildet und weist an dem Ende, das mit dem Atemschlauch 4 verbunden ist, ein Entnahmeröhrchen 16 auf, das ein Analysegas aus der Atemluft des Patienten entnimmt. Das Analysegas wird durch die Gasprobenleitung 12 zu einem Gasanalysator 22, beispielsweise einem Kapnograph, gefördert. Dieser weist in der Regel eine Pumpe auf, die das Analysegas durch die Gasprobenleitung 12 ansaugt.

Die Gasprobenleitung 12 weist einen weiteren Anschluss 18 zum Anschluss an den Gasanalysator auf. Ebenfalls kann kurz vor Eintritt des Analysegases in den Gasanalysator 22 eine Wasserfalle 20 an der Gasprobenleitung 12 angeordnet sind, um Wassertröpfchen vom Detektor fernzuhalten.

Die Gasprobenleitung 12 selbst besteht aus einem atmungsaktiven Copolymer-Material, nämlich einem so genannten thermoplastischen Polyether-ester (TPEE) bzw. Polyether-ester Blockcopolymer, das eine hohe Wasserdampftransportrate aufweist. Dieses Material ist kostengünstig und lässt sich sehr einfach in verschiedenen Formen extrudieren oder spritzgießen, unter anderem auch als Schlauch. Entsprechende Materialien sind in einer breiten Bandbreite von mechanischen Eigenschaften kommerziell erhältlich, beispielsweise unter dem Markennamen Hytrel^{®} von DuPont. Geometrie, Wanddicke, Schlauchlänge und Material lassen sich so auswählen, dass ein für die Zwecke einer Gasprobenleitung mit zu trocknendem Analysegas geeigneter Schlauch mit ausreichend hoher Wasserdampftransportrate entsteht. So wird eine geringere Wanddicke zu einer höheren Wasserdampftransportrate führen, wodurch kürzere Schläuche möglich werden, falls dies gewünscht ist. Zur Wahrung der Stabilität des Schlauches sollte die Wanddicke allerdings eine materialspezifische Untergrenze nicht unterschreiten.

Bei einem Seitenstromverfahren, bei dem beispielsweise 70 bis 200 ml pro Minute der Atemluft eines Patienten abgeführt wird, kommt es zu einem Kondensatanfall von ca. 1,5 bis 5 g Wasser pro 24 Stunden, der durch den Schlauch in die Umgebungsluft abgeführt werden soll. Weist dieser eine Länge von 3.000 mm und einen Innendurchmesser von 1 mm auf, so bedeutet dies, dass er einen Wert der Wasserdampftransportrate (MVTR) von ca. 150 bis 500 g/m²/24h oder mehr aufweisen sollte.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein.

### Bezugszeichenliste

- 2: Beatmungsgerät
- 4: Atemschlauch
- 6: Atemmaske
- 10: Gasanalysesystem
- 12: Gasprobenleitung
- 14: Anschluss
- 16: Entnahmeröhrchen
- 18: Anschluss
- 20: Wasserfalle
- 22: Gasanalysator

## Patentansprüche

1. Gasprobenleitung (12) für ein Analysegas, insbesondere Atemgas, zur Leitung eines Analysesgases von einer Analysegasquelle (4) zu einem Gasanalysator (22), die mit einer Analysegasquelle (4) und einem Gasanalysator (22) verbindbar ist, **dadurch gekennzeichnet, dass** die Gasprobenleitung (12) als Schlauch aus einem thermoplastischen Polyether-ester Blockcopolymer-Material ausgebildet ist.

2. Gasprobenleitung (12) nach Anspruch 1, **dadurch gekennzeichnet, dass** das Polyether-ester Blockcopolymer harte Segmente aus Polybutylenterephthalat und weiche Segmente aus einem Polyetherglykol aufweist.

3. Gasprobenleitung (12) nach Anspruch 1 oder 2, **dadurch gekennzeichnet, dass** die Polyethersegmente und die Polyestersegmente des Polyether-ester Blockcopolymers in einem Verhältnis von zwischen 25:75 und 75:25, insbesondere zwischen 40:60 und 60:40, auftreten.

4. Gasprobenleitung (12) nach einem der Ansprüche 1 bis 3, **dadurch gekennzeichnet, dass** der Schlauch bei 38°C und 90% relativer Luftfeuchtigkeit eine Wasserdampftransmissionsrate von mehr als 300 g/m²/24h, insbesondere mehr als 1.000 g/m²/24h, aufweist.

5. Gasprobenleitung (12) nach einem der Ansprüche 1 bis 4, **dadurch gekennzeichnet, dass** das Polyether-ester Blockcopolymer eine Shore-D-Härte zwischen 30 und 85, insbesondere zwischen 35 und 60, aufweist.

6. Gasprobenleitung (12) nach einem der Ansprüche 1 bis 5, **dadurch gekennzeichnet, dass** der Schlauch eine Länge von 0,5 bis 5 m aufweist, insbesondere 2 bis 4 m, insbesondere zwischen 2 m und 3 m.

7. Gasprobenleitung (12) nach einem der Ansprüche 1 bis 6, **dadurch gekennzeichnet, dass** der Schlauch einen Innendurchmesser zwischen 0,25 und 2 mm, insbesondere zwischen 0,5 und 1 mm, sowie eine Wandstärke von 0,2 mm bis 1,0 mm, insbesondere von 0,4 bis 0,6 mm, aufweist.

8. Gasanalysesystem (10) mit einem Gasanalysator (22), insbesondere Kapnograph, sowie einer Gasprobenleitung (12) nach einem der Ansprüche 1 bis 7.

9. Verwendung einer Gasprobenleitung (12) nach einem der Ansprüche 1 bis 7 zum Vortrocknen eines Analysegases für eine Gasanalyse, das durch die Gasprobenleitung (12) zu einem Gasanalysator (22), insbesondere Kapnograph, geleitet wird.

10. Verfahren zum Analysieren eines Analysegases, wobei eine Probe eines Analysegases aus einem Analysegasstrom entnommen und durch eine Gasprobenleitung (12) nach einem der Ansprüche 1 bis 7 vorgetrocknet und zu einem Gasanalysator (22) geleitet wird.
